# FASCICULE DE BREVET EUROPEEN

(11) **EP 1 066 281 B1**
(45) Date de publication et mention de la délivrance du brevet: **31.10.2001**
(21) Numéro de dépôt: 00900584.4
(22) Date de dépôt: 14.01.2000
(51) Int. Cl.: C07D 405/12, C07D 317/66, A61K 7/13

(54) **METHYLENEDIOXY-BENZENES CATIONIQUES, LEUR UTILISATION POUR LA TEINTURE D'OXYDATION DES FIBRES KERATINIQUES, COMPOSITIONS ET PROCEDE DE TEINTURE**
KATIONISCHE METHYLENDIOXY-BENZOLE, DEREN VERWENDUNG ZUM OXIDATIVEN FÄRBEN KERATINISCHER FASERN, FÄRBEMITTEL UND FÄRBEVERFAHREN
CATIONIC METHYLENEDIOXY BENZENES, THEIR USE FOR OXIDATION DYEING OF KERATIN FIBRES, DYEING COMPOSITIONS AND METHODS

(30) Priorité: 21.01.1999 FR 9900635
(43) Date de publication de la demande: 10.01.2001
(73) Titulaire: L'OREAL, 75008 Paris (FR)
(72) Inventeur: GENET, Alain, F-93600 Aulnay-sous-Bois (FR); LAGRANGE, Alain, F-77700 Coupvray (FR)
(74) Mandataire: Miszputen, Laurent
(86) Numéro de dépôt international: FR0000076
(87) Numéro de publication internationale: WO0043388

(56) Documents cités:
- EP-A- 0 424 525
- FR-A- 2 586 913
- FR-A- 2 603 483
- US-A- 4 975 092

## Description

L'invention a pour objet de nouveaux méthylènedioxy-benzènes comportant au moins un groupement cationique Z, Z étant choisi parmi des chaînes aliphatiques quatemisées, des chaînes aliphatiques comportant au moins un cycle saturé quaternisé, et des chaînes aliphatiques comportant au moins un cycle insaturé quaternisé, leur utilisation à titre de précurseur de colorant d'oxydation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que par exemples des coupleurs indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir, de façon totalement inattendue et surprenante, qu'une nouvelle famille de méthylènedioxy-benzènes de formule (I) ci-après définie, comportant au moins un groupement cationique Z, Z étant choisi parmi des chaînes aliphatiques quaternisées, des- chaînes aliphatiques comportant au moins un cycle saturé quaternisé, et des chaînes aliphatiques comportant au moins un cycle insaturé quatemisé, conviennent pour une utilisation comme précurseur de colorant d'oxydation pour la coloration d'oxydation, mais en outre qu'ils permettent d'obtenir des compositions tinctoriales conduisant à des colorations puissantes, dans une très large palette de nuances et présentant d'excellentes propriétés de résistance aux différents traitements que peuvent subir les fibres kératiniques. Enfin, ces composés s'avèrent être aisément synthétisables.

Ces découvertes sont à la base de la présente invention.

L'invention a donc pour premier objet des méthylènedioxy-benzènes de formule (I) suivante, et leurs sels d'addition avec un acide : 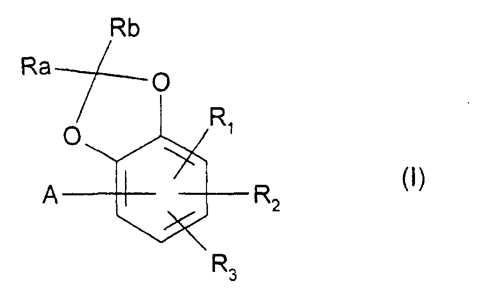 dans laquelle :
- Ra et Rb, identiques ou différents, peuvent représenter un atome d'hydrogène, un radical alkyle en C₁-C₆, un radical hydroxyalkyle en C₁-C₆, ou forment, conjointement avec l'atome dé carbone auquel ils sont rattachés, un cycle carboné saturé à 5, 6 ou 7 chaînons ;
- R₁, R₂ et R₃, qui peuvent être identiques ou différents, représentent un atome d'hydrogène ; un atome d'halogène ; un groupement Z tel que défini ci-après ; un groupement A' tel que défini ci-après ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₈)carbonyle ; un radical aminoalkyl(C₁-C₆)carbonylalkyle(C₁-C₆) ; un radical N-Z-aminoalkyl (C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z définis ci-après, ou pouvant former ensemble, avec l'atome d'azote auquel ils sont rattachés, un cycle à 5 ou 6 chaînons, carboné, ou contenant un ou plusieurs hétéroatomes ;
- R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'alkyle est substitué ou non substitué par un ou plusieurs radicaux hydroxy et dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, et parmi les groupements Z ; ou pouvant formant ensemble avec l'atome d'azote auquel ils sont rattachés, un cycle à 5 ou 6 chaînons carboné ou contenant un ou plusieurs hétéroatomes ;
   - A représente un groupement -NR₄R₅ ou un radical hydroxyle ;
   - A' représente un groupement -NR'₄R'₅ ou un radical hydroxyle ;
- R₄, R₅, R'₄ et R'₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par ou plusieurs radicaux hydroxy; un radical aminoalkyle en C₁-C₆ dont l'alkyle est substitué ou non substitué par ou plusieurs radicaux hydroxy et dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle, N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z, ou pouvant former ensemble avec l'atome d'azote auquel ils sont rattachés, un cycle à 5 ou 6 chaînons carboné ou contenant un ou plusieurs hétéroatomes ;
- Z est choisi parmi les groupements cationiques insaturés de formules (II) et (III) suivantes, et les groupements cationiques saturés de formule (IV) suivante : 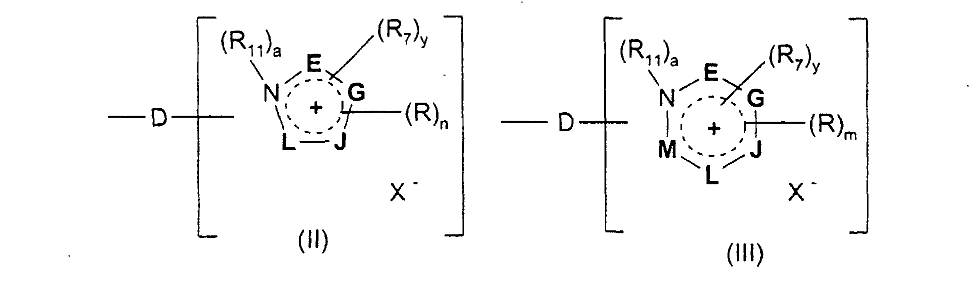 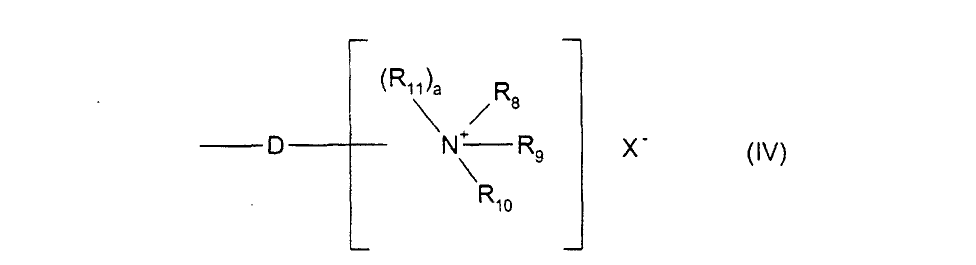 dans lesquelles :
   - D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement ;
   - les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₇ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un second groupement Z identique ou différent du premier groupement Z ;
- R₈, R₉ et R₁₀, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₈, R₉ et R₁₀ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
   l'un des radicaux R₈, R₉ et R₁₀ peut également représenter un second groupement Z, identique ou différent du premier groupement Z ;
- R₁₁ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- a et y sont des nombres entiers égaux à 0 ou 1 ; avec les conditions suivantes :
   - dans les groupements cationiques insaturés de formule (II) :
      - lorsque a = 0, le bras de liaison D est rattaché à l'atome d'azote,
      - lorsque a = 1, le bras de liaison D est rattaché à l'un des sommets E, G, J ou L,
      - y ne peut prendre la valeur 1 que :
         1) lorsque les sommets E, G, J et L représentent simultanément un atome de carbone, et que le radical R₇ est porté par l'atome d'azote du cycle insaturé ; ou bien
         2) lorsqu'au moins un des sommets E, G, J et L représente un atome d'azote sur lequel le radical R₇ est fixé ;
   - dans les groupements cationiques insaturés de formule (III) :
      - lorsque a = 0, le bras de liaison D est rattaché à l'atome d'azote,
      - lorsque a = 1, le bras de liaison D est rattaché à l'un des sommets E, G, J, L ou M,
      - y ne peut prendre la valeur 1 que lorsqu'au moins un des sommets E, G, J, L et M représente un atome divalent, et que le radical R₇ est porté par l'atome d'azote du cycle insaturé ;
   - dans les groupements cationiques de formule (IV)
      - lorsque a = 0, alors le bras de liaison D est rattaché à l'atome d'azote portant les radicaux R₈ à R₁₀,
      - lorsque a = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
- X représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
étant entendu que :
- le nombre de groupement cationique Z est au moins égal à 1.

Comme indiqué précédemment, les colorations obtenues avec la composition de teinture d'oxydation conforme à l'invention sont puissantes et permettent d'atteindre des nuances dans une très large palette de couleurs. Elles présentent de plus d'excellentes propriétés de résistance vis à vis de l'action des différents agents extérieurs (lumière, intempéries, lavage, ondulation permanente, transpiration, frottements). Ces propriétés sont particulièrement remarquables notamment en ce qui concerne la résistance des colorations obtenues vis à vis de l'action de la lumière, des lavages, et de la transpiration.

Dans les formules (I), (II), (III) et (IV) ci-dessus les radicaux alkyle et alcoxy peuvent être linéaires ou ramifiés.

Parmi les cycles carbonés pouvant être formés conjointement par les radicaux Ra et Rb, on peut particulièrement citer les cycles pentane, hexane et heptane.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Les composés de formule (I) ci-dessus, sont de préférence choisis parmi :
- le chlorure de 1-[2-(benzo[1,3]dioxol-5-ylamino)-éthyl]-3-méthyl-3H-imidazol-1-ium ;
- le chlorure de 1-[2-(6-hydroxy-benzo[1,3]dioxol-5-ylamino)-éthyl]-3-méthyl-3H-imidazol-1-ium ;
- le chlorure de 1-[2-(6-méthoxy-benzo[1,3]dioxol-5-ylamino)-éthyl]-3-méthyl-3H-imidazol-1-ium ;
- le chlorure de 1-[2-(6-éthoxy-benzo[1,3]dioxol-5-ylamino)-éthyl]-3-méthyl-3H-imidazol-1-ium ;
- le chlorure de 1-{2-[6-(2-hydroxy-éthoxy)-benzo[1,3]dioxol-5-ylamino]-éthyl}-3-méthyl-3H-imidazol-1-ium ;
- le chlorure de 1-[2-(6-amino-benzo[1,3]dioxol-5-ylarnino)-éthyl]-3-méthyl-3H-imidazol-1-ium ;
- le dichlorure de 1-{2-[6-(2-(3-méthyl-3H-imidazol-1-ium)-éthoxy)-benzo[1,3]dioxol-5-ylamino]-éthyl)-3-méthyl-3H-imidazol-1-ium ;
- le chlorure de 1-[2-(6-amino-benzo[1,3]dioxol-5-yloxy)-éthyl]-3-méthyl-3H-imidazol-1-ium ;
- le chlorure de 1-[3-(6-amino-benzo[1,3]dioxol-5-yloxy)-propyl]-3-méthyl-3H-imidazol-1-ium ;
- le bromure de [2-(6-amino-benzo[1,3]dioxol-5-yloxy)-éthyl]-diéthyl-(2-hydroxyéthyl)-ammonium ;
- le méthylsulfate de [2-(6-amino-benzo[1,3]dioxol-5-yloxy)-éthyl]-diéthyl-méthylammonium ;
- le chlorure de 1-[2-(6-amino-benzo[1,3]dioxol-5-yloxy)-éthyl]-1,4-diméthylpipérazin-1-ium ;
- le chlorure de 1-[2-(2,2-bis-hydroxyméthyl-6-méthoxy-benzo[1,3]dioxol-5-ylamino)-éthyl]-3-méthyl-3H-imidazol-1-ium ;
et leurs sels d'addition avec un acide.

Les sels d'addition avec un acide des composés de formule (I) conformes à l'invention sont de préférence choisis parmi les chlorhydrates, les bromhydrates, les sulfates, les citrates, les succinates, les tartrates, les lactates et les acétates.

Les composés de formule (I) conformes à l'invention peuvent être facilement obtenus, selon des méthodes bien connues de l'état de la technique, par exemple par réduction des composés nitrés cationiques correspondants lorsque ces composés comportent un groupement amino.

Cette étape de réduction (obtention d'une amine aromatique primaire) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de formylation, de benzènesulfonation, etc...), faire ensuite la ou les substitutions ou modifications désirées (y compris la quaternisation) et terminer par la "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déprotection" en milieu acide).

Les chaînes cationiques sont elles mêmes obtenues par des méthodes bien connues de l'état de la technique.

L'obtention des amines quaternisées, peut par exemple être réalisée :
- en un temps, par condensation d'un composé méthylènedioxy-benzénique comportant un radical halogénoalkyle avec un composé porteur d'un radical amine tertiaire, ou par condensation d'un composé méthylènedioxy-benzénique comportant un radical amine tertiaire avec un composé porteur d'un radical halogénoalkyle, ou par quaternisation avec un agent alkylant d'un composé méthylènedioxy-benzénique comportant un radical amine tertiaire ;
- ou en deux temps, par condensation d'un composé méthylènedioxybenzénique comportant un radical halogénoalkyle avec un composé porteur d'une amine secondaire, suivie d'une quaternisation avec un agent alkylant.

Les radicaux halogénoalkyle portés par les composés méthylènedioxybenzéniques intermédiaires peuvent être préparés par des méthodes connues de l'état de la technique, en une ou plusieurs étapes, par exemple par condensation d'un composé dihalogénoalkyle sur une amine ou un hydroxyle, ou par halogénation d'une chaîne hydroxyalkyle.

Lorsque la synthèse est terminée, les composés de formule (I) conformes à l'invention peuvent, le cas échéant, être récupérés par des méthodes bien connues de l'état de la technique telles que la cristallisation, la distillation.

Un autre objet de l'invention est l'utilisation des composés de formule (I) conformes à l'invention à titre de précurseur de colorant d'oxydation pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de colorant d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 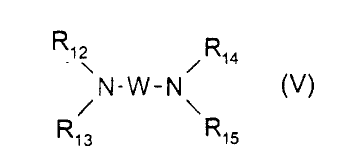 dans laquelle W est un reste propylène éventuellement substitué par un groupement hydroxyle ou un radical alkyle en C₁-C₆ ; R₁₂, R₁₃, R₁₄ et R₁₅, identiques ou différents, représentent un atome d'hydrogène, un radical alkyle en C₁-C₆ ou hydroxyalkyle en C₁-C₆.

La composition tinctoriale conforme à l'invention peut encore contenir, en plus du ou des composés de formule (I) définie ci-dessus, au moins une base d'oxydation qui peut être choisie parmi les bases d'oxydation classiquement utilisées en teinture d'oxydation et parmi lesquelles on peut notamment citer les paraphénylènediamines, les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyethyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène-diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les ortho-aminophénols, on peut plus particulièrement citer à titre d'exemple, citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo-[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs formes tautomères, lorsqu'il existe un équilibre tautomérique, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer un ou plusieurs coupleurs et/ou un ou plusieurs colorants directs, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre de l'invention (composés de formule (I), bases d'oxydation et coupleurs) sont notamment choisis parmi les chlorhydrates, les bromhydrates, les sulfates, les citrates, les succinates, les tartrates, les lactates et les acétates.

La composition tinctoriale conforme l'invention peut également renfermer divers adjuvants utilisés classiquement dans les compositions pour la teinture des cheveux, tels que des agents tensio-actifs anioniques, cationiques, nonioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, et notamment lorsque la composition tinctoriale conforme à l'invention renferme une ou plusieurs bases d'oxydation et/ou un ou plusieurs coupleurs, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLE DE PREPARATION

### EXEMPLE DE PREPARATION 1 : Synthèse du chlorure 1-[2-(6-aminobenzo[1,3]dioxol-5-ylamino)-éthyl]-3-méthyl-3H-imidazol-1-ium, chlorhydrate.

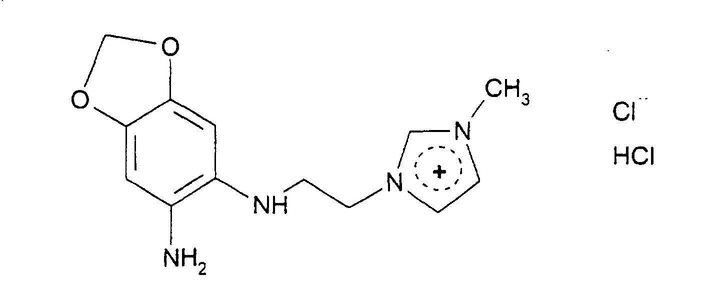

### a) Préparation de la (2-chloro-éthyl)-(6-nitro-benzo[1,3]dioxol-5-yl)-amine

On a fait une solution de 50,0 g (0,22 mole) de 2-(6-nitro-benzo[1,3]dioxol-5-ylamino)-éthanol (RN 106146-44-5) et de 42 ml de triéthylamine dans 260 ml de diméthylformamide que l'on a refroidie à une température d'environ 0°C.
On a coulé goutte à goutte en 40 minutes, et en maintenant la température entre 0 et 5°C, 20,4 ml (0,263 mole) de chlorure de mésyle.
On a laissé remonter la température vers 20°C et ajouté 27,8 g (0,658 mole) de chlorure de lithium (la réaction était exothermique).
On a chauffé pendant une demi-heure au bain marie bouillant et versé dans 750 g d'eau glacée.
Le précipité cristallisé a été essoré, réempaté dans l'eau et séché.
Après recristallisation de l'acétate d'éthyle au reflux, on a obtenu 38,9 g de cristaux orangés de (2-chloro-éthyl)-(6-nitro-benzo[1,3]dioxol-5-yl)-amine qui ont fondu à 120°C (Kofler) et dont l'analyse élémentaire calculée pour C₉H₉N₂O₄Cl était :

| % | C | H | N | O | Cl |
|---|---|---|---|---|---|
| Calculé | 44,19 | 3,71 | 11,45 | 26,16 | 14,49 |
| Trouvé | 44,31 | 3,70 | 11,68 | 26,40 | 14,30 |

### b) Préparation du chlorure de 3-méthyl-1-[2-(6-nitro-benzo[1,3]dioxol-5-ylamino)-éthyl]-3H-imidazol-1-ium

On a chauffé au reflux pendant 8 heures un mélange de 48,9 g (0,2 mole) de (2-chloro-éthyl)-(6-nitro-benzo[1,3]dioxol-5-yl)-amine obtenu ci-dessus à l'étape précédente et de 49,3 g (0,6 mole) de 1-méthyl-1H-imidazole dans 100 ml de toluène.
On a refroidit et essoré le précipité cristallisé.
Après purification par recristallisation d'un mélange d'éthanol à 96° et d'eau au reflux, on a obtenu 52,7g de cristaux orangés de chlorure de 3-méthyl-1-[2-(6-nitro-benzo[1,3]dioxol-5-ylamino)-éthyl]-3H-imidazol-1-ium qui ont fondu à une température de 203°C (Kofler) et dont l'analyse élémentaire calculée pour C₁₃H₁₅N₄O₄Cl + ½ H₂O était :

| % | C | H | N | O | Cl |
|---|---|---|---|---|---|
| Calculé | 46,51 | 4,80 | 16,69 | 21,44 | 10,56 |
| Trouvé | 46,22 | 4,78 | 15,98 | 21,76 | 10,59 |

### c) Réduction du chlorure de 3-méthyl-1-[2-(6-nitro-benzo[1,3]dioxol-5-ylamino)-éthyl]-3H-imidazol-1 -ium

Dans un hydrogénateur, on a placé 52,0 g (0,159 mole) du composé obtenu ci-dessus à l'étape précédente, 15 g de palladium à 5% sur charbon (contenant 50% d'eau), 300 ml d'éthanol 96° et 300 ml d'eau.
La réduction s'est faite en une ½ heure sous une pression d'hydrogène d'environ 8 bars et à une température qui a progressivement été portée à 70°C.

Après filtration du catalyseur sous azote, on a coulé sur 60 ml d'acide chlorhydrique à 36% et évaporé le filtrat à sec sous pression réduite. Le composé a été repris plusieurs fois dans l'éthanol absolu. Après recristallisation d'un mélange éthanol/eau au reflux et séchage à 40°C sous vide et sur potasse, on a obtenu 29,3 g de cristaux légèrement gris de chlorure 1-[2-(6-amino-benzo[1,3]dioxol-5-ylamino)-éthyl]-3-méthyl-3H-imidazol-1-ium, chlorhydrate, qui ont fondu avec décomposition à 238-240°C (Kofler) et dont l'analyse élémentaire calculée pour C₁₃H₁₈N₄O₂Cl₂ était :

| % | C | H | N | O | Cl |
|---|---|---|---|---|---|
| Calculé | 46,86 | 5,44 | 16,81 | 9,60 | 21,28 |
| Trouvé | 46,59 | 5,44 | 16,26 | 10,15 | 21,38 |

### EXEMPLES D'APPLICATION

### EXEMPLE 1 DE TEINTURE A L'AIR

On a préparé, au moment de l'emploi, la composition tinctoriale conforme à l'invention suivante :
- Chlorure 1-[2-(6-amino-benzo[1,3]dioxol-5-ylamino)-éthyl]-3-méthyl-3H-imidazol-1-ium, chlorhydrate (composé de formule (I)) 1,0 g
- Ethanol à 96° 20 g
- Tampon pH 9,5 NH₄OH/NH₄Cl (1M/1M) 10 g
- Eau déminéralisée qsp 100 g

Cette composition a été appliquée sur des mèches de cheveux gris permanentés à 90% de blancs, et on laissé la coloration se développer pendant 30 minutes, sans ajout d'agent oxydant autre que l'oxygène de l'air.

Les cheveux ont ensuite été rincés, lavés avec un shampooing standard, rincés à nouveau puis séchés.

Les cheveux ont été teints dans une nuance blond cuivré irisé.

### EXEMPLE 2 DE TEINTURE EN MILIEU NEUTRE

On a préparé la composition tinctoriale conforme à l'invention suivante :
- Chlorure 1-[2-(6-amino-benzo[1,3]dioxol-5-ylamino)-éthyl]-3-méthyl-3H-imidazol-1-ium, chlorhydrate (composé de formule (I)) 1,0 g
- Ethanol à 96° 18 g - Tampon K₂HPO₄ /KH₂PO₄ (1,5 M / 1 M) 10 g
- Métabisulfite de sodium 0,68 g
- Sel pentasodique de l'acide diéthylènetriaminopentacétique 1,1 g
- Eau déminéralisée qsp 100 g

Au moment de l'emploi, on a mélangé poids pour poids la composition tinctoriale ci-dessus avec une solution de peroxyde d'hydrogène à 20 volumes (6 % en poids) de pH 3.

Le mélange obtenu a été appliqué sur des mèches de cheveux gris permanentés à 90 % de blancs pendant 30 minutes. Les mèches ont ensuite été rincées, lavées avec un shampooing standard, rincées à nouveau puis séchées.

Les cheveux ont été teints dans une nuance blond irisé légèrement doré.

### EXEMPLE 3 DE TEINTURE ENZYMATIQUE

On a préparé la composition tinctoriale prête à l'emploi suivante :
- Chlorure 1-[2-(6-amino-benzo[1,3]dioxol-5-ylamino)-éthyl]-3-méthyl-3H-imidazol-1-ium, chlorhydrate (composé de formule (I)) 1,0 g
- Ethanol à 96° 10 g
- Uricase d'Arthrobacter Globiformis à 20 Unités Internationales (U.I.) / mg, commercialisée par la société SIGMA 1,0 g
- Acide urique 1,0 g
- Monoéthanolamine q.s. pH = 9,5
- Eau déminéralisée q.s.p. 100,0 g

La composition tinctoriale prête à l'emploi décrite ci-dessus a été appliquée sur des mèches de cheveux gris naturels à 90 % de blancs pendant 30 minutes. Les cheveux ont ensuite été rincés, lavés avec un shampooing standard, puis séchés.

Les cheveux ont été teints dans un nuance blond cuivré irisé.

## Revendications

1. Composés de formule (I) suivante, et leurs sels d'addition avec un acide : 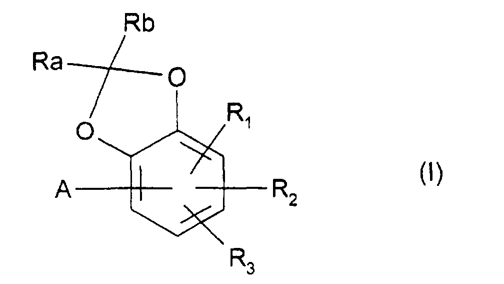 dans laquelle :
• Ra et Rb, identiques ou différents, peuvent représenter un atome d'hydrogène, un radical alkyle en C₁-C₆, un radical hydroxyalkyle en C₁-C₆, ou forment, conjointement avec l'atome de carbone auquel ils sont rattachés, un cycle carboné saturé à 5, 6 ou 7 chaînons ;
• R₁, R₂ et R₃, qui peuvent être identiques ou différents, représentent un atome d'hydrogène ; un atome d'halogène ; un groupement Z ; un groupement A' ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle un radical aminosulfonylalkyle(C₁-C₆) ; un radical N-Z-amino sulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un radical aminoalkyle en C₁-C₆ dont l'alkyle est substitué ou non substitué par un ou plusieurs radicaux hydroxy ; un radical aminoalkyle(C₁-C₆) dont l'alkyle est substitué ou non substitué par un ou plusieurs radicaux hydroxy et dont l'amine est substituée par un ou deux radicaux, identiques ou différents, choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z, ou pouvant former ensemble, avec l'atome d'azote auquel ils sont rattachés, un cycle à 5 ou 6 chaînons, carboné, ou contenant un ou plusieurs hétéroatomes ;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'alkyle est substitué ou non substitué par un ou plusieurs radicaux hydroxy et dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, et parmi les groupements Z ; ou pouvant formant ensemble avec l'atome d'azote auquel ils sont rattachés, un cycle à 5 ou 6 chaînons carboné ou contenant un ou plusieurs hétéroatomes ;
• A représente un groupement -NR₄R₅ ou un radical hydroxyle ;
• A' représente un groupement -NR'₄R'₅ ou un radical hydroxyle ;
• R₄, R₅, R'₄ et R'₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ dont l'alkyle est substitué ou non substitué par ou plusieurs radicaux hydroxy; un radical aminoalkyle en C₁-C₆ dont l'alkyle est substitué ou non substitué par ou plusieurs radicaux hydroxy et dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle, N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou parmi les groupements Z, ou pouvant former ensemble avec l'atome d'azote auquel ils sont rattachés, un cycle à 5 ou 6 chaînons carboné ou contenant un ou plusieurs hétéroatomes ;
• Z est choisi parmi les groupements cationiques insaturés de formules (II) et (III) suivantes, et les groupements cationiques saturés de formule (IV) suivante : 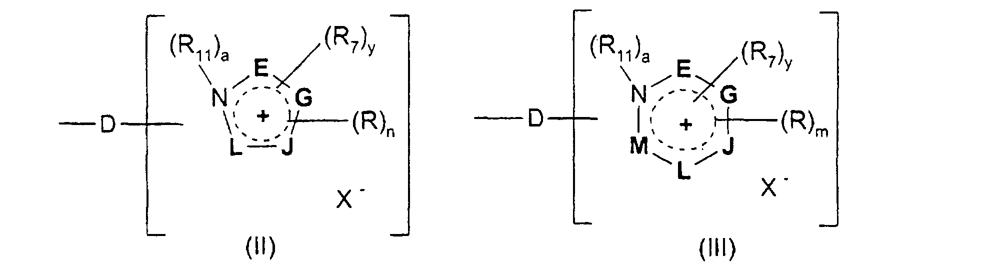 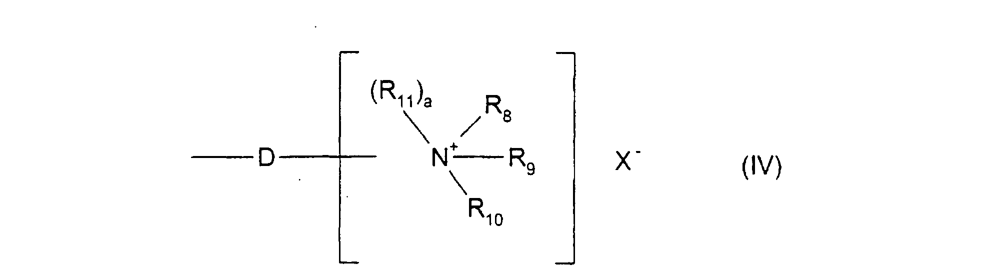 dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₇ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, ou un second groupement Z identique ou différent du premier groupement Z ;
• R₈, R₉ et R₁₀, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₈, R₉ et R₁₀ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₈, R₉ et R₁₀ peut également représenter un second groupement Z, identique ou différent du premier groupement Z ;
• R₁₁ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• a et y sont des nombres entiers égaux à 0 ou 1 ; avec les conditions suivantes :
- dans les groupements cationiques insaturés de formule (II) :
- lorsque a = 0, le bras de liaison D est rattaché à l'atome d'azote,
- lorsque a = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
- dans les groupements cationiques insaturés de formule (III) :
- lorsque a = 0, le bras de liaison D est rattaché à l'atome d'azote,
- lorsque a = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
- dans les groupements cationiques de formule (IV) :
- lorsque a = 0, alors le bras de liaison D est rattaché à l'atome d'azote portant les radicaux R₈ à R₁₀,
- lorsque a = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
• X représente un anion monovalent ou divalent ;
étant entendu que:
- le nombre de groupement cationique Z est au moins égal à 1.

2. Composés selon la revendication 1, **caractérisés par le fait que** les cycles des groupements insaturés Z de formule (II) sont choisis parmi les cycles pyrrolique, imidazolique, pyrazolique, oxazolique, thiazolique et triazolique.

3. Composés selon la revendication 1, **caractérisés par le fait que** les cycles des groupements insaturés Z de formule (III) sont choisis parmi les cycles pyridinique, pyrimidinique, pyrazinique, oxazinique et triazinique.

4. Composés selon l'une quelconque des revendications précédentes, **caractérisés par le fait que** deux des R₈, R₉ et R₁₀ forment un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₈)sulfonyle.

5. Composés selon l'une quelconque des revendications précédentes, **caractérisés par le fait que** X représente un atome d'halogène, un hydroxyde, un hydrogènesulfate, ou un alkyl(C₁-C₆)sulfate.

6. Composés selon l'une quelconque des revendications précédentes, **caractérisés par le fait qu'**ils sont choisis parmi :
- le chlorure de 1-[2-(benzo[1,3]dioxol-5-ylamino)-éthyl]-3-méthyl-3H-imidazol-1-ium ;
- le chlorure de 1-[2-(6-hydroxy-benzo[1,3]dioxol-5-ylamino)-éthyl]-3-méthyl-3H-imidazol-1-ium ;
- le chlorure de 1-[2-(6-méthoxy-benzo[1,3]dioxol-5-ylamino)-éthyl]-3-méthyl-3H-imidazol-1-ium ;
- le chlorure de 1-[2-(6-éthoxy-benzo[1,3]dioxol-5-ylamino)-éthyl]-3-méthyl-3H-imidazol-1-ium ;
- le chlorure de 1-{2-[6-(2-hydroxy-éthoxy)-benzo[1,3]dioxol-5-yiamino]-éthyl}-3-méthyl-3H-imidazol-1-ium ;
- le chlorure de 1-[2-(6-amino-benzo[1,3]dioxol-5-ylamino)-éthyl]-3-méthyl-3H-imidazol-1-ium ;
- le dichlorure de 1-{2-[6-(2-(3-méthyl-3H-imidazol-1-ium)-éthoxy)-benzo[1,3]dioxol-5-ylamino]-éthyl}-3-méthyl-3H-imidazol-1-ium ;
- le chlorure de 1-[2-(6-amino-benzo[1,3]dioxol-5-yloxy)-éthyl]-3-méthyl-3H-imidazol-1-ium ;
- le chlorure de 1-[3-(6-amino-benzo[1,3]dioxol-5-yloxy)-propyl]-3-méthyl-3H-imidazol-1-ium ;
- le bromure de [2-(6-amino-benzo[1,3]dioxol-5-yloxy)-éthyl]-diéthyl-(2-hydroxyéthyl)-ammonium ;
- le méthylsulfate de [2-(6-amino-benzo[1,3]dioxol-5-yloxy)-éthyl]-diéthyl-méthylammonium ;
- le chlorure de 1-[2-(6-amino-benzo[1,3]dioxol-5-yloxy)-éthyl]-1,4-diméthylpipérazin-1-ium ;
- le chlorure de 1-[2-(2,2-bis-hydroxyméthyl-6-méthoxy-benzo[1,3]dioxol-5-ylamino)-éthyl]-3-méthyl-3H-imidazol-1-ium ;
et leurs sels d'addition avec un acide.

7. Composés selon l'une quelconque des revendications précédentes, **caractérisés par le fait que** les sels d'addition avec un acide sont choisis parmi les chlorhydrates, les bromhydrates, les sulfates, les citrates, les succinates, les tartrates, les lactates et les acétates.

8. Utilisation des composés de formule (I) tels que définis à l'une quelconque des revendications 1 à 7, à titre de précurseur de colorant d'oxydation pour la teinture d'oxydation des fibres kératiniques.

9. Composition pour la teinture d'oxydation des fibres kératiniques, **caractérisée par le fait qu'**elle comprend, dans un milieu approprié pour la teinture, au moins un composé de formule (I) tel que défini à l'une quelconque des revendications 1 à 7.

10. Composition selon la revendication 9, **caractérisée par le fait que** le ou les composés de formule (I) représentent de 0,0005 à 12 % en poids du poids total de la composition tinctoriale.

11. Composition selon la revendication 10, **caractérisée par le fait que** le ou les composés de formule (I) représentent de 0,005 à 6 % en poids du poids total de la composition tinctoriale.

12. Composition selon l'une quelconque des revendications 9 à 11, **caractérisée par le fait qu'**elle renferme une ou plusieurs bases d'oxydation choisies parmi les paraphénylènediamines, les bis-phénylalkylènediamines, les para-aminophénols, les ortho-aminophénols et les bases hétérocycliques.

13. Composition selon la revendication 12, **caractérisée par le fait que** les bases hétérocycliques sont choisies parmi les dérivés pyridiniques, les dérivés pyrimidiniques et les dérivés pyrazoliques.

14. Composition selon la revendication 12 ou 13, **caractérisée par le fait que** la ou les bases d'oxydation représentent de 0,0005 à 12 % en poids du poids total de la composition tinctoriale.

15. Composition selon l'une quelconque des revendications 9 à 14, **caractérisée par le fait qu'**elle renferme un ou plusieurs coupleurs choisis parmi les métaphénylènediamines, les méta-aminophénols, les métadiphénols et les coupleurs hétérocycliques, et/ou un ou plusieurs colorants directs.

16. Composition selon la revendication 15, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

17. Composition selon la revendication 15 ou 16, **caractérisée par le fait que** le ou les coupleurs représentent de 0,0001 à 10 % en poids environ du poids total de la composition tinctoriale.

18. Composition selon l'une quelconque des revendications 9 à 17, **caractérisée par le fait que** les sels d'addition avec un acide sont choisis parmi les chlorhydrates, les bromhydrates, les sulfates, les citrates, les succinates, les tartrates, les lactates et les acétates.

19. Procédé de teinture d'oxydation des fibres kératiniques et en particulier des fibres kératiniques humaines telles que les cheveux, **caractérisé par le fait qu'**on applique sur les fibres au moins une composition tinctoriale telle que définie à l'une quelconque des revendications 9 à 18, pendant un temps suffisant pour développer la coloration désirée, soit à l'air, soit à l'aide d'un agent oxydant.

20. Procédé selon la revendication 19, **caractérisé par le fait que** la coloration des fibres peut être effectuée sans addition d'un agent oxydant, au seul contact de l'oxygène de l'air.

21. Procédé selon la revendication 19, **caractérisé par le fait qu'**on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

22. Procédé selon la revendication 21, **caractérisé par le fait que** l'agent oxydant est choisi parmi le peroxyde d'hydrogène, le peroxyde d'urée, les bromates de métaux alcalins, les persels et les enzymes.

23. Procédé selon la revendication 22, **caractérisé par le fait que** les enzymes sont choisies parmi les peroxydases, les laccases, les tyrosynases et les oxydo-réductases.

24. Procédé selon la revendication 23, **caractérisé par le fait que** les oxydo-réductases sont choisies parmi les pyranose oxydases, les glucose oxydases, les glycérol oxydases, les lactates oxydases, les pyruvate oxydases, et les uricases.

25. Dispositif à plusieurs compartiments, ou "kit" de teinture à plusieurs compartiments, dont un premier compartiment renferme une composition tinctoriale telle que définie à l'une quelconque des revendications 9 à 18 et un second compartiment renferme une composition oxydante.

## Patentansprüche

1. Verbindungen der folgenden Formel (I) und ihre Additionssalze mit einer Säure: 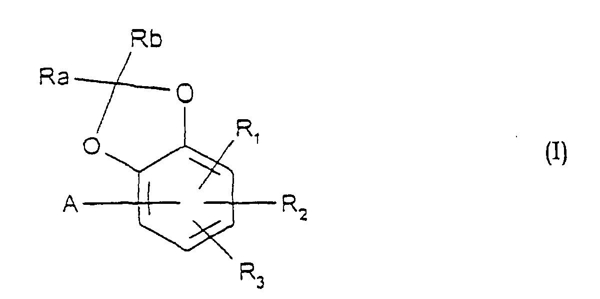 worin bedeuten:
• Ra und Rb, die identisch oder voneinander verschieden sind, können ein Wasserstoffatom, eine C₁₋₆-Alkylgruppe oder eine C₁₋₆-Hydroxyalkylgruppe bedeuten oder sie bilden gemeinsam mit dem Kohlenstoffatom, an das sie gebunden sind, einen gesättigten, 5-, 6- oder 7-gliedrigen Kohlenstoffring;
• R₁, R₂ und R₃, die identisch oder voneinander verschieden sein können, bedeuten Wasserstoff; Halogen; eine Gruppe Z; eine Gruppe A'; Alkyl(C₁₋₆)carbonyl; Aminoalkyl(C₁₋₆)carbonyl; N-Z-Aminoalkyl(C₁₋₆)carbonyl; N-Alkyl(C₁₋₆)aminoalkyl(C₁₋₆)-carbonyl; N,N-Dialkyl(C₁₋₆)aminoalkyl(C₁₋₆)carbonyl; Aminoalkyl(C₁₋₆)carbonylalkyl(C₁₋₆); N-Z-Aminoalkyl(C₁₋₆)carbonylalkyl(C₁₋₆); N-Alkyl(C₁₋₆)aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl(C₁₋₆)sulfonyl; Aminosulfonyl; N-Z-Aminosulfonyl; N-Alkyl(C₁₋₆)aminosulfonyl; N,N-Dialkyl(C₁₋₆)aminosulfonyl; Aminosulfonylalkyl(C₁₋₆); N-Z-Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); C₁₋₆-Alkyl; C₁₋₆-Monohydroxyalkyl; C₂₋₆-Polyhydroxyalkyl; Alkoxy(C₁₋₆)alkyl(C₁₋₆); C₁₋₆-Trifluoralkyl; Cyano; OR₆ oder SR₆; C₁₋₆-Aminoalkyl, wobei die Alkylgruppe unsubstituiert vorliegt oder mit einer oder mehreren Hydroxygruppen substituiert ist; Aminoalkyl(C₁₋₆), wobei die Alkylgruppe unsubstituiert vorliegt oder mit einer oder mehreren Hydroxygruppen substituiert ist und wobei die Aminogruppe mit einer oder zwei Gruppen substituiert ist, die identisch oder voneinander verschieden sind und die unter den folgenden Gruppen ausgewählt sind: Alkyl, C₁₋₆-Monohydroxyalkyl, C₂₋₆-Polyhydroxyalkyl, Alkyl(C₁₋₆)carbonyl, Carbamoyl, N-Alkyl(C₁₋₆)carbamoyl oder N,N-Dialkyl(C₁₋₆)-carbamoyl, Alkyl(C₁₋₆)sulfonyl, Formyl, Trifluoralkyl(C₁₋₆)-carbonyl, Alkyl(C₁₋₆)carboxy, Thiocarbamoyl oder den Gruppen Z, oder sie können gemeinsam mit dem Stickstoffatom, an das sie gebunden sind, einen 5- oder 6-gliedrigen Ring bilden, der Kohlenstoffatome enthält oder auch ein oder mehrere Heteroatome aufweist;
• R₆ bedeutet C₁₋₆-Alkyl; C₁₋₆-Monohydroxyalkyl; C₂₋₆-Polyhydroxyalkyl; eine Gruppe Z; Alkoxy(C₁₋₆)alkyl(C₁₋₆); Aryl; Benzyl; C₁₋₆-Carboxyalkyl; Alkyl(C₁₋₆)carboxyalkyl(C₁₋₆); C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Trifluoralkyl; C₁₋₆-Aminosulfonylalkyl; N-Z-C₁₋₆-Aminosulfonylalkyl; N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); C₁₋₆-Aminoalkyl; C₁₋₆-Aminoalkyl, wobei die Alkylgruppe unsubstituiert vorliegt oder mit einer oder mehreren Hydroxygruppen substituiert ist und wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Formyl, Trifluoralkyl(C₁₋₆)-carbonyl, Alkyl(C₁₋₆)carboxy, Carbamoyl, N-Alkyl(C₁₋₆)-carbamoyl, N,N-Dialkyl(C₁₋₆)carbamoyl, Thiocarbamoyl, Alkyl-(C₁₋₆)sulfonyl oder den Gruppen Z; oder sie können gemeinsam mit dem Stickstoffatom, an das sie gebunden sind, einen 5- oder 6-gliedrigen Ring bilden, der Kohlenstoffatome enthält oder auch ein oder mehrere Heteroatome aufweist;
• A bedeutet eine Gruppe -NR₄R₅ oder eine Hydroxygruppe;
• A' bedeutet eine Gruppe -NR'₄R'₅ oder eine Hydroxygruppe;
• R₄, R₅, R'₄ und R'₅, die identisch oder voneinander verschieden sind, bedeuten Wasserstoff; eine Gruppe Z; C₁₋₆-Alkyl; C₁₋₆-Monohydroxyalkyl; C₂₋₆-Polyhydroxyalkyl; Alkoxy(C₁₋₆)-alkyl(C₁₋₆); Aryl; Benzyl; C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Thiocarbamoylalkyl; C₁₋₆-Trifluoralkyl; C₁₋₆-Sulfoalkyl; Alkyl(C₁₋₆)carboxyalkyl(C₁₋₆); Alkyl(C₁₋₆)sulfinylalkyl(C₁₋₆); C₁₋₆-Aminosulfonylalkyl; N-Z-C₁₋₆-Aminosulfonylalkyl; N-Alkyl(C₁₋₆)aminosulfonylalkyl(C₁₋₆); N,N-Dialkyl(C₁₋₆)aminosulfonylalkyl(C₁₋₆); Alkyl(C₁₋₆)carbonylalkyl(C₁₋₆); C₁₋₆-Aminoalkyl, wobei die Alkylgruppe unsubstituiert vorliegt oder mit einer oder mehreren Hydroxygruppen substituiert ist; C₁₋₆-Aminoalkyl, wobei die Alkylgruppe unsubstituiert vorliegt oder mit einer oder mehreren Hydroxygruppen substituiert ist und wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Carbamoyl, N-Alkyl(C₁₋₆)-carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy, Thiocarbamoyl oder den Gruppen Z; oder sie können gemeinsam mit dem Stickstoffatom, an das sie gebunden sind, einen 5-oder 6-gliedrigen Ring bilden, der Kohlenstoffatome enthält oder ein oder mehrere Heteroatome aufweist;
• Z ist unter den ungesättigten kationischen Gruppen der folgenden Formeln (II) und (III) und den gesättigten kationischen Gruppen der folgenden Formel (IV) ausgewählt: 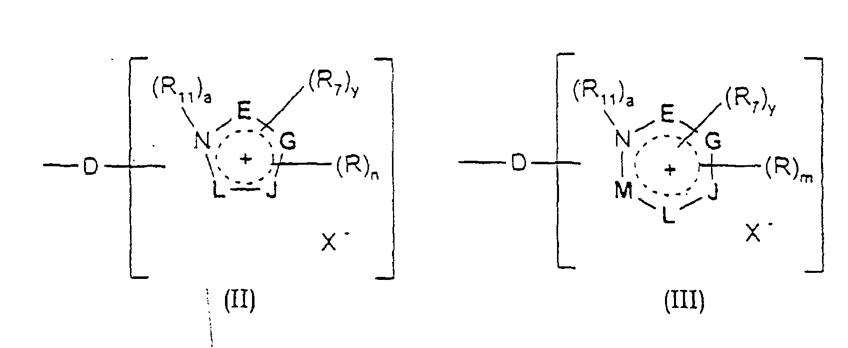 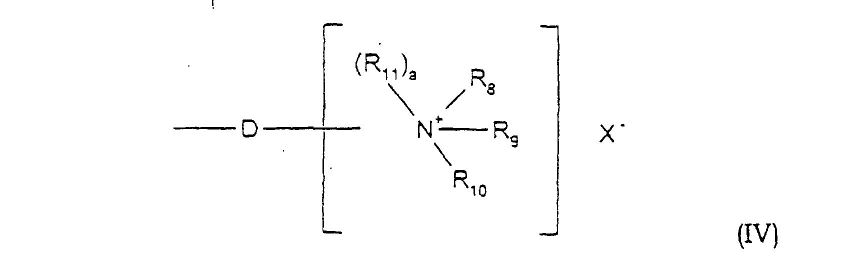 worin bedeuten:
• D eine Verbindungsgruppe, die eine Alkylkette mit vorzugsweise 1 bis 14 Kohlenstoffatome bedeutet, die geradkettig oder verzweigt vorliegt und mit einem oder mehreren Heteroatomen, wie beispielsweise Sauerstoff, Schwefel oder Stickstoff, unterbrochen sein kann, mit einer oder mehreren Hydroxygruppen oder C₁₋₆-Alkoxygruppen substituiert sein kann und eine oder mehrere Ketogruppen tragen kann;
• E, G, J, L und M, die identisch oder voneinander verschieden sind, bedeuten ein Kohlenstoffatom, ein Sauerstoffatom, ein Schwefelatom oder ein Stickstoffatom;
• n ist 0 oder eine ganze Zahl von 1 bis 4;
• m ist 0 oder eine ganze Zahl von 1 bis 5;
• die Gruppen R, die identisch oder voneinander verschieden sind, bedeuten eine zweite Gruppe Z, die identisch mit der ersten Gruppe Z oder von ihr verschieden sein kann, Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Alkylcarbonyl, Thio, C₁₋₆-Thioalkyl, Alkyl(C₁₋₆)thio, eine Aminogruppe, die mit Alkyl(C₁₋₆)carbonyl, Carbamoyl oder Alkyl(C₁₋₆)sulfonyl geschützt ist; eine Gruppe NHR" oder NR"R"', worin R" und R"', die identisch oder voneinander verschieden sind, C₁₋₆-Alkyl, C₁₋₆-Monohydroxyalkyl oder C₂₋₆-Polyhydroxyalkyl bedeuten;
• R₇ bedeutet C₁₋₆-Alkyl, C₁₋₆-Monohydroxyalkyl, C₂₋₆-Polyhydroxyalkyl, C₁₋₆-Cyanoalkyl, Trialkyl(C₁₋₆)silanalkyl(C₁₋₆), Alkoxy(C₁₋₆)alkyl(C₁₋₆), C₁₋₆-Carbamoylalkyl, Alkyl(C₁₋₆)-carboxyalkyl(C₁₋₆), Benzyl oder eine zweite Gruppe Z, die identisch mit der ersten Gruppe Z oder von ihr verschieden ist;
• R₈, R₉ und R₁₀, die identisch oder voneinander verschieden sind, C₁₋₆-Alkyl, C₁₋₆-Monohydroxyalkyl, C₂₋₆-Polyhydroxyalkyl, Alkoxy(C₁₋₆)alkyl(C₁₋₆), C₁₋₆-Cyanoalkyl, Aryl, Benzyl, C₁₋₆-Amidoalkyl, Trialkyl(C₁₋₆)silanalkyl(C₁₋₆) oder C₁₋₆-Aminoalkyl, wobei die Aminogruppe mit Alkyl(C₁₋₆)carbonyl, Carbamoyl oder Alkyl(C₁₋₆)sulfonyl geschützt ist; zwei der Gruppen R₈, R₉ und R₁₀ können auch gemeinsam mit dem Stickstoffatom, an das sie gebunden sind, einen 5- oder 6-gliedrigen Ring bilden, der Kohlenstoffatome enthält oder ein oder mehrere Heteroatome aufweist, wobei der Ring unsubstituiert vorliegen kann oder substituiert sein kann mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)-silanalkyl(C₁₋₆), Amido, Aldehydo, Carboxy, C₁₋₆-Ketoalkyl, Thio, C₁₋₆-Thioalkyl, Alkyl(C₁₋₆)thio, Amino, einer Aminogruppe, die mit Alkyl(C₁₋₆)carbonyl, Carbamoyl oder Alkyl(C₁₋₆)sulfonyl geschützt ist;
wobei eine der Gruppen R₈, R₉ und R₁₀ eine zweite Gruppe Z bedeuten kann, die identisch mit der ersten Gruppe Z oder von ihr verschieden ist;
• R₁₁ bedeutet C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, wobei die Aminogruppe mit Alkyl(C₁₋₆)carbonyl, Carbamoyl oder Alkyl(C₁₋₆)sulfonyl geschützt ist; C₁₋₆-Carboxyalkyl; C₁₋₆-Cyanoalkyl; C₁₋₆-Carbamoylalkyl; C₁₋₆-Trifluoralkyl; Trialkyl(C₁₋₆)silanalkyl(C₁₋₆); C₁₋₆-Sulfonamidoalkyl; Alkyl(C₁₋₆)carboxyalkyl(C₁₋₆); Alkyl(C₁₋₆)sulfinylalkyl(C₁-₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
• a und y bedeuten 0 oder 1; mit den folgenden Bedingungen:
- in den ungesättigten kationischen Gruppen der Formel (II):
- die Verbindungsgruppe D ist an das Stickstoffatom gebunden, wenn a = 0.
- die Verbindungsgruppe D ist an eines der Atome E, G, J oder L gebunden, wenn a = 1,
- y kann nur den Wert 1 annehmen, wenn:
1) die Gruppe R₇ von dem Stickstoffatom des ungesättigten Rings getragen wird, wenn die Atome E, G, J und L gleichzeitig ein Kohlenstoffatom bedeuten; oder
2) mindestens eines der Atome E, G, J und L ein Stickstoffatom bedeutet, an das R₇ gebunden ist;
- in den ungesättigten kationischen Gruppen der Formel (III):
- die Verbindungsgruppe D an das Stickstoffatom gebunden ist, wenn a = 0,
- die Verbindungsgruppe D an eines der Atome E, G, J, L oder M gebunden ist, wenn a = 1,
- y nur den Wert 1 annehmen kann, wenn mindestens eines der Atome E, G, J, L und M ein zweiwertiges Atom bedeutet und die Gruppe R₇ von dem Stickstoffatom des ungesättigten Rings getragen wird;
- in den kationischen Gruppen der Formel (IV):
- die Verbindungsgruppe D an das Stickstoffatom gebunden ist, das die Gruppen R₈ bis R₁₀ trägt, wenn a = 0,
- zwei der Gruppen R₈ bis R₁₀ gemeinsam mit dem Stickstoffatom, an das sie gebunden sind, einen oben definierten gesättigten 5- oder 6-gliedrigen Ring bilden und die Verbindungsgruppe D von einem Kohlenstoffatom des gesättigten Rings getragen wird, wenn a = 1;
• X⁻ bedeutet ein einwertiges oder zweiwertiges Anion; mit der Maßgabe, daß:
- die Anzahl der kationischen Gruppen Z mindestens 1 ist.

2. Verbindungen nach Anspruch 1, **dadurch gekennzeichnet, daß** die Ringe der ungesättigten Gruppen Z der Formel (II) unter Pyrrolringen, Imidazolringen, Pyrazolringen, Oxazolringen, Thiazolringen und Triazolringen ausgewählt sind.

3. Verbindungen nach Anspruch 1, **dadurch gekennzeichnet, daß** die Ringe der ungesättigten Gruppen Z der Formel (III) unter den Pyridinringen, Pyrimidinringen, Pyrazinringen, Oxazinringen und Triazinringen ausgewählt sind.

4. Verbindungen nach einem der vorhergehenden Ansprüche, **dadurch gekennzeichnet, daß** zwei der Gruppen R₈, R₉ und R₁₀ einen Pyrrolidinring, Piperidinring, Piperazinring oder Morpholinring bilden, wobei der Ring unsubstituiert vorliegen oder mit einem Halogenatom, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Ketoalkyl, Thio, C₁₋₆-Thioalkyl, Alkyl(C₁₋₆)thio, Amino oder einer Aminogruppe substituiert sein kann, die mit Alkyl(C₁₋₆)-carbonyl, Carbamoyl oder Alkyl(C₁₋₆)sulfonyl geschützt sein kann.

5. Verbindungen nach einem der vorhergehenden Ansprüche, **dadurch gekennzeichnet, daß** X⁻ ein Halogenatom, ein Hydroxid, ein Hydrogensulfat oder ein Alkyl(C₁₋₆)sulfat bedeutet.

6. Verbindungen nach einem der vorhergehenden Ansprüche, **dadurch gekennzeichnet, daß** sie ausgewählt sind unter:
- 1-[2-(Benzo[1,3]dioxol-5-ylamino)-ethyl]-3-methyl-3H-imidazol-1-ium-chlorid;
- 1-[2-(6-Hydroxy-benzo [1,3]dioxol-5-ylamino)-ethyl]-3-methyl-3H-imidazol-1-ium-chlorid;
- 1-[2-(6-Methoxy-benzo[1,3]dioxol-5-ylamino)-ethyl]-3-methyl-3H-imidazol-1-ium-chlorid;
- 1-[2-(6-Ethoxy-benzo[1,3]dioxol-5-ylamino)-ethyl]-3-methyl-3H-imidazol-1-ium-chlorid;
- 1-{2-[6-(2-Hydroxy-ethoxy)-benzo[1,3]dioxol-5-ylamino]-ethyl}-3-methyl-3H-imidazol-1-ium-chlorid;
- 1-[2-(6-Amino-benzo[1,3]dioxol-5-ylamino)-ethyl]-3-methyl-3H-imidazol-1-ium-chlorid;
- 1-{2-[6-(2-(3-Methyl-3H-imidazol-1-ium)-ethoxy)-benzo[1,3]-dioxol-5-ylamino)-ethyl}-3-methyl-3H-imidazol-1-iumdichlorid;
- 1-[2-(6-Amino-benzo[1,3]dioxol-5-yloxy)-ethyl]-3-methyl-3H-imidazol-1-ium-chlorid;
- 1-[3-(6-Amino-benzo[1,3]dioxol-5-yloxy)-propyl]-3-methyl-3H-imidazol-1-ium-chlorid;
- [2-(6-Amino-benzo[1,3]dioxol-5-yloxy)-ethyl]-diethyl-(2-hydroxy-ethyl)-ammonium-bromid;
- [2-(6-Amino-benzo[1,3]dioxol-5-yloxy)-ethyl]-diethyl-methylammonium-methylsulfat;
- 1-[2-(6-Amino-benzo[1,3]dioxol-5-yloxy)-ethyl]-1,4-dimethylpiperazin-1-ium-chlorid;
- 1-[2-(2,2-Bis-hydroxymethyl-6-methoxy-benzo[1,3]dioxol-5-ylamino)-ethyl]-3-methyl-3H-imidazol-1-ium-chlorid;
und ihren Additionssalzen mit einer Säure.

7. Verbindungen nach einem der vorhergehenden Ansprüche, **dadurch gekennzeichnet, daß** die Additionssalze mit einer Säure unter den Hydrochloriden, Hydrobromiden, Sulfaten, Citraten, Succinaten, Tartraten, Lactaten und Acetaten ausgewählt sind.

8. Verwendung von Verbindungen der Formel (I) nach einem der Ansprüche 1 bis 7 als Farbstoffvorprodukte von Oxidationsfarbstoffen zum oxidativen Färben von Keratinfasern.

9. Zusammensetzung zum oxidativen Färben von Keratinfasern, **dadurch gekennzeichnet, daß** sie in einem zum Färben geeigneten Medium mindestens eine Verbindung der Formel (I) nach einem der Ansprüche 1 bis 7 enthält.

10. Zusammensetzung nach Anspruch 9, **dadurch gekennzeichnet, daß** die Verbindung(en) der Formel (I) 0,0005 bis 12 Gew.-% des Gesamtgewichts der Farbmittelzusammensetzung ausmachen.

11. Zusammensetzung nach Anspruch 10, **dadurch gekennzeichnet, daß** die Verbindung(en) der Formel (I) 0,005 bis 6 Gew.-% des Gesamtgewichts der Farbmittelzusammensetzung ausmachen.

12. Zusammensetzung nach einem der Ansprüche 9 bis 11, **dadurch gekennzeichnet, daß** sie eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

13. Zusammensetzung nach Anspruch 12, **dadurch gekennzeichnet, daß** die heterocyclischen Basen unter den Pyridinderivaten, Pyrimidinderivaten und Pyrazolderivaten ausgewählt sind.

14. Zusammensetzung nach Anspruch 12 oder 13, **dadurch gekennzeichnet, daß** die Oxidationsbase(n) 0,0005 bis 12 Gew.-% des Gesamtgewichts der Farbmittelzusammensetzung ausmachen.

15. Zusammensetzung nach einem der Ansprüche 9 bis 14, **dadurch gekennzeichnet, daß** sie einen oder mehrere Kuppler, die unter den m-Phenylendiaminen, m-Aminophenolen, m-Dihydroxybenzolen und heterocyclischen Kupplern ausgewählt sind, und/oder einen oder mehrere Direktfarbstoffe enthält.

16. Zusammensetzung nach Anspruch 15, **dadurch gekennzeichnet, daß** die Kuppler unter 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis-(2,4-diaminophenoxy)-propan, Sesamol, α-Naphthol, 6-Hydroxyindol, 4-Hydroxyindol, 4-Hydroxy-N-methylindol, 6-Hydroxyindolin, 2,6-Dihydroxy-4-methylpyridin, 1-H-3-Methyl-pyrazol-5-on, 1-Phenyl-3-methyl-pyrazol-5-on und deren Additionssalzen mit einer Säure ausgewählt sind.

17. Zusammensetzung nach einem der Ansprüche 15 oder 16, **dadurch gekennzeichnet, daß** der oder die Kuppler etwa 0,0001 bis 10 Gew.-% des Gesamtgewichts der Farbmittelzusammensetzung ausmachen.

18. Zusammensetzung nach einem der Ansprüche 9 bis 17, **dadurch gekennzeichnet, daß** die Additionssalze mit einer Säure unter den Hydrochloriden, Hydrobromiden, Sulfaten, Citraten, Succinaten, Tartraten, Lactaten und Acetaten ausgewählt sind.

19. Verfahren zum oxidativen Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 9 bis 18 während einer Zeitspanne aufgebracht wird, die ausreichend ist, um die gewünschte Färbung entweder mit Luftsauerstoff oder einem Oxidationsmittel zu entwickeln.

20. Verfahren nach Anspruch 19, **dadurch gekennzeichnet, daß** die Färbung der Fasern ohne Zusatz eines Oxidationsmittels nur im Kontakt mit Luftsauerstoff erfolgen kann.

21. Verfahren nach Anspruch 19, **dadurch gekennzeichnet, daß** die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgebracht wird.

22. Verfahren nach Anspruch 21, **dadurch gekennzeichnet, daß** das Oxidationsmittel unter Wasserstoffperoxid, Harnstoffperoxid, Alkalimetallbromaten, Salzen von Persäuren und Enzymen ausgewählt ist.

23. Verfahren nach Anspruch 22, **dadurch gekennzeichnet, daß** die Enzyme unter den Peroxidasen, Laccasen, Tyrosinasen und Oxidoreduktassen ausgewählt sind.

24. Verfahren nach Anspruch 23, **dadurch gekennzeichnet, daß** die Oxidoreduktasten unter den Pyranoseoxydasen, Glucoseoxydasen, Glycerinoxydasen, Lactatoxydasen, Pyruvatoxydasen und Uricasen ausgewählt sind.

25. Vorrichtung mit mehreren Abteilungen oder "Kit" zum Färben mit mehreren Abteilungen, wobei eine erste Abteilung eine Farbmittelzusammensetzung nach einem der Ansprüche 9 bis 18 und eine zweite Abteilung eine oxidierende Zusammensetzung enthält.

## Claims

1. Compounds of formula (I) below, and the addition salts thereof with an acid: 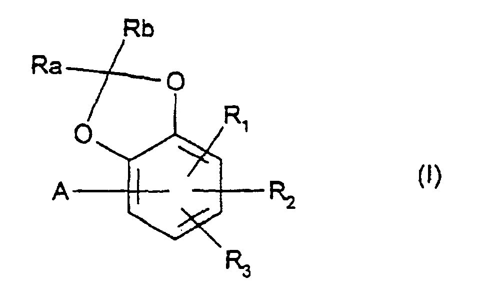 in which:
• Ra and Rb, which may be identical or different, can represent a hydrogen atom, a C₁-C₆ alkyl radical, a C₁-C₆ hydroxyalkyl radical or form, together with the carbon atom to which they are attached, a saturated 5-, 6- or 7-membered carbon-based ring;
• R₁, R₂ and R₃, which may be identical or different, represent a hydrogen atom; a halogen atom; a group Z; a group A'; a (C₁-C₆)alkylcarbonyl radical; an amino (C₁-C₆) alkylcarbonyl radical; an N-Z-amino(C₁-C₆)alkylcarbonyl radical; an N- (C₁-C₆) alkylamino (C₁-C₆) alkylcarbonyl radical; an N,N-di (C₁-C₆) alkylamino (C₁-C₆) alkylcarbonyl radical; an amino (C₁-C₆) alkylcarbonyl (C₁-C₆) alkyl radical; an N-Z-amino (C₁-C₆) alkylcarbonyl (C₁-C₆) alkyl radical; an N-(C₁-C₆)alkylamino(C₁-C₆)alkylcarbonyl(C₁-C₆)-alkyl radical; an N,N-di(C₁-C₆)alkylamino(C₁-C₆)-alkylcarbonyl(C₁-C₆)alkyl radical; a carboxyl radical; a (C₁-C₆)alkylcarboxyl radical; a C₁-C₆ alkylsulphonyl radical; an aminosulphonyl radical; an N-Z-aminosulphonyl radical; a C₁-C₆ N-alkylaminosulphonyl radical; an N,N-di(C₁-C₆)-alkylaminosulphonyl radical; a C₁-C₆ aminosulphonylalkyl radical; a C₁-C₆ N-Z-aminosulphonylalkyl radical; an N-(C₁-C₆)alkylaminosulphonyl(C₁-C₆)alkyl radical; an N,N-di(C₁-C₆)-alkylaminosulphonyl(C₁-C₆)alkyl radical; a carbamyl radical; an N-(C₁-C₆)alkylcarbamyl radical; an N,N-di(C₁-C₆)alkylcarbamyl radical; a carbamyl-(C₁-C₆)alkyl radical; an N-(C₁-C₆)alkylcarbamyl-(C₁-C₆)alkyl radical; an N,N-di(C₁-C₆)alkylcarbamyl-(C₁-C₆)alkyl radical; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a cyano radical; a group OR₆ or SR₆; a C₁-C₆ aminoalkyl radical in which the alkyl is unsubstituted or substituted with one or more hydroxyl radicals; an amino(C₁-C₆)alkyl radical in which the alkyl is unsubstituted or substituted with one or more hydroxyl radicals and in which the amine is substituted with one or two identical or different radicals chosen from alkyl, C₁-C₆ monohydroxyalkyl, C₂-C₆ polyhydroxyalkyl, (C₁-C₆)alkylcarbonyl, carbamyl, N-(C₁-C₆)alkylcarbamyl or N,N-di(C₁-C₆)-alkylcarbamyl, (C₁-C₆)alkylsulphonyl, formyl, trifluoro(C₁-C₆)alkylcarbonyl, (C₁-C₆)alkylcarboxyl and thiocarbamyl radicals, or from the groups Z, or which are able to form, with the nitrogen atom to which they are attached, a 5- or 6-membered carbon-based ring or a ring containing one or more hetero atoms;
• R₆ denotes a C₁-C₆ alkyl radical; a C₁-C₆ monohydroxyalkyl radical; a C₂-C₆ polyhydroxyalkyl radical; a group Z; a (C₁-C₆)alkoxy(C₁-C₆)alkyl radical; an aryl radical; a benzyl radical; a carboxy(C₁-C₆)alkyl radical; a (C₁-C₆)alkylcarboxy-(C₁-C₆)alkyl radical; a cyano(C₁-C₆)alkyl radical; a carbamyl(C₁-C₆)alkyl radical; an N-(C₁-C₆)-alkylcarbamyl(C₁-C₆)alkyl radical; an N,N-di(C₁-C₆)-alkylcarbamyl(C₁-C₆)alkyl radical; a C₁-C₆ trifluoroalkyl radical; a C₁-C₆ aminosulphonylalkyl radical; a C₁-C₆ N-Z-aminosulphonylalkyl radical; an N-(C₁-C₆)alkylaminosulphonyl(C₁-C₆)alkyl radical; an N,N-di(C₁-C₆)alkylaminosulphonyl(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the alkyl is unsubstituted or substituted by one or more hydroxyl radicals and in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, formyl, trifluoro-(C₁-C₆)alkylcarbonyl, (C₁-C₆)alkylcarboxyl, carbamyl, N-(C₁-C₆)alkylcarbamyl, N,N-di-(C₁-C₆)-alkylcarbamyl, thiocarbamyl and C₁-C₆ alkylsulphonyl radicals, and from the groups Z; or which are able to form, together with the nitrogen atom to which they are attached, a 5- or 6-membered carbon-based ring or a ring containing one or more hetero atoms;
• A represents a group -NR₄R₅ or a hydroxyl radical;
• A' represents a group -NR'₄R'₅ or a hydroxyl radical;
• R₄, R₅, R'₄ and R'₅, which may be identical or different, represent a hydrogen atom; a group Z; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; an aryl radical; a benzyl radical; a cyano(C₁-C₆)alkyl radical; a carbamyl (C₁-C₆) alkyl radical; an N-(C₁-C₆)alkylcarbamyl(C₁-C₆)alkyl radical; an N,N-di(C₁-C₆)-alkylcarbamyl(C₁-C₆)alkyl radical; a thiocarbamyl-(C₁-C₆)alkyl radical; a C₁-C₆ trifluoroalkyl radical; a C₁-C₆ sulphoalkyl radical; a (C₁-C₆)-alkylcarboxy (C₁-C₆) alkyl radical; a (C₁-C₆)alkylsulphinyl (C₁-C₆) alkyl radical; a C₁-C₆ aminosulphonylalkyl radical; a C₁-C₆ N-Z-aminosulphonylalkyl radical; an N-(C₁-C₆)alkylaminosulphonyl (C₁-C₆) alkyl radical; an N,N-di(C₁-C₆)-alkylaminosulphonyl(C₁-C₆)alkyl radical; a (C₁-C₆)-alkylcarbonyl(C₁-C₆)alkyl radical; a C₁-C₆ aminoalkyl radical in which the alkyl is unsubstituted or substituted with one or more hydroxyl radicals; a C₁-C₆ aminoalkyl radical in which the alkyl is unsubstituted or substituted with one or more hydroxyl radicals and in which the amine is substituted with one or two identical or different radicals chosen from C₁-C₆ alkyl, C₁-C₆ monohydroxyalkyl, C₂-C₆ polyhydroxyalkyl, (C₁-C₆)-alkylcarbonyl, carbamyl, N-(C₁-C₆)alkylcarbamyl, N,N-di(C₁-C₆)alkylcarbamyl, (C₁-C₆)alkylsulphonyl, formyl, trifluoro(C₁-C₆)alkylcarbonyl, (C₁-C₆)alkylcarboxyl and thiocarbamyl radicals, or from the groups Z, or which are able to form, together with the nitrogen atom to which they are attached, a 5-or 6-membered carbon-based ring or a ring containing one or more hetero atoms;
• Z is chosen from the unsaturated cationic groups of formulae (II) and (III) below, and the saturated cationic groups of formula (IV) below: 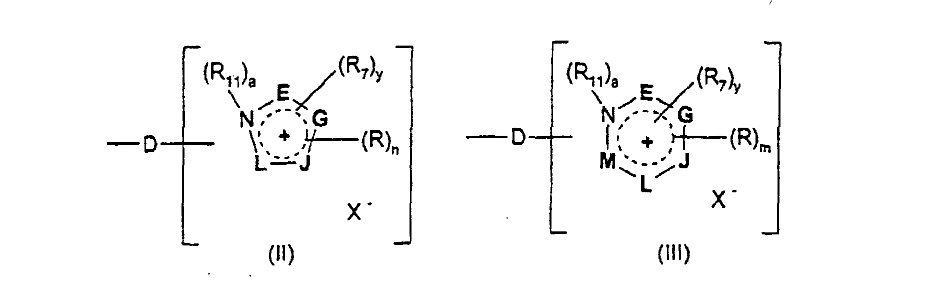 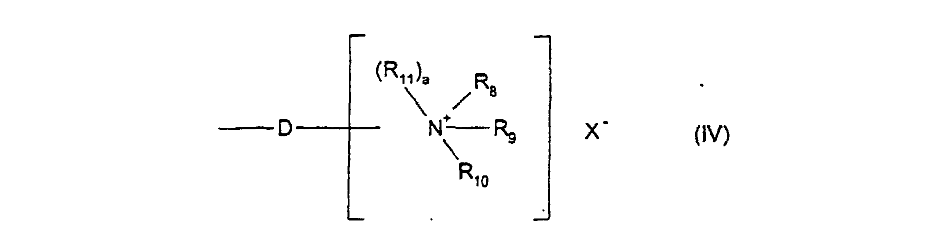 in which:
• D is a linker arm which represents a linear or branched alkyl chain preferably containing from 1 to 14 carbon atoms, which can be interrupted by one or more hetero atoms such as oxygen, sulphur or nitrogen atoms, and which can be substituted with one or more hydroxyl or C₁-C₆ alkoxy radicals, and which can bear one or more ketone functions;
• the ring members E, G, J, L and M, which may be identical or different, represent a carbon, oxygen, sulphur or nitrogen atom;
• n is an integer between 0 and 4 inclusive;
• m is an integer between 0 and 5 inclusive;
• the radicals R, which may be identical or different, represent a second group Z which is identical to or different from the first group Z, a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano (C₁-C₆) alkyl radical, a C₁-C₆ alkoxy radical, a tri (C₁-C₆) alkylsilane (C₁-C₆) alkyl radical, an amido radical, an aldehydo radical, a carboxyl radical, a (C₁-C₆)alkylcarbonyl radical, a thio radical, a C₁-C₆ thioalkyl radical, a C₁-C₆ alkylthio radical, an amino radical, an amino radical protected with a (C₁-C₆)alkylcarbonyl, carbamyl or C₁-C₆ alkylsulphonyl radical; a group NHR'' or NR''R''' in which R" and R''', which may be identical or different, represent a C₁-C₆ alkyl radical, a C₁-C₆ monohydroxyalkyl radical or a C₂-C₆ polyhydroxyalkyl radical;
• R₇ represents a C₁-C₆ alkyl radical, a C₁-C₆ monohydroxyalkyl radical, a C₂-C₆ polyhydroxyalkyl radical, a cyano(C₁-C₆)alkyl radical, a tri-(C₁-C₆) alkylsilane (C₁-C₆) alkyl radical, a (C₁-C₆)-alkoxy (C₁-C₆) alkyl radical, a carbamyl-(C₁-C₆)alkyl radical, a (C₁-C₆)alkylcarboxy(C₁-C₆)-alkyl radical, a benzyl radical or a second group Z which is identical to or different from the first group Z;
• R₈, R₉ and R₁₀, which may be identical or different, represent a C₁-C₆ alkyl radical, a C₁-C₆ monohydroxyalkyl radical, a C₂-C₆ polyhydroxyalkyl radical, a (C₁-C₆)alkoxy(C₁-C₆)alkyl radical, a cyano(C₁-C₆)alkyl radical, an aryl radical, a benzyl radical, a C₁-C₆ amidoalkyl radical, a tri(C₁-C₆)alkylsilane(C₁-C₆)alkyl radical or a C₁-C₆ aminoalkyl radical in which the amine is protected with a (C₁-C₆)alkylcarbonyl, carbamyl or C₁-C₆ alkylsulphonyl radical; two of the radicals R₈, R₉ and R₁₀ can together also form, with the nitrogen atom to which they are attached, a saturated 5- or 6-membered carbon-based ring or a ring containing one or more hetero atoms, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri (C₁-C₆) alkylsilane (C₁-C₆) alkyl radical, an amido radical, an aldehydo radical, a carboxyl radical, a keto (C₁-C₆)alkyl radical, a thio radical, a C₁-C₆ thioalkyl radical, a C₁-C₆ alkylthio radical, an amino radical or an amino radical protected with a (C₁-C₆)alkylcarbonyl, carbamyl or C₁-C₆ alkylsulphonyl radical;
one of the radicals R₈, R₉ and R₁₀ can also represent a second group Z which is identical to or different from the first group Z;
• R₁₁ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is protected with a (C₁-C₆)-alkylcarbonyl, carbamyl or C₁-C₆ alkylsulphonyl radical; a carboxy(C₁-C₆)alkyl radical; a cyano-(C₁-C₆)alkyl radical; a carbamyl(C₁-C₆)alkyl radical; a C₁-C₆ trifluoroalkyl radical; a tri-(C₁-C₆)alkylsilane(C₁-C₆)alkyl radical; a C₁-C₆ sulphonamidoalkyl radical; a (C₁-C₆)alkylcarboxy-(C₁-C₆)alkyl radical; a (C₁-C₆)alkylsulphinyl-(C₁-C₆)alkyl radical; a (C₁-C₆)alkylsulphonyl-(C₁-C₆)alkyl radical; a (C₁-C₆)alkylketo(C₁-C₆)alkyl radical; an N-(C₁-C₆)alkylcarbamyl(C₁-C₆)alkyl radical; an N-(C₁-C₆)alkylsulphonamido(C₁-C₆)alkyl radical;
• a and y are integers equal to 0 or 1; with the following conditions:
- in the unsaturated cationic groups of formula (II):
- when a = 0, the linker arm D is attached to the nitrogen atom,
- when a = 1, the linker arm D is attached to one of the ring members E, G, J or L,
- y can take the value 1 only:
1) when the ring members E, G, J and L simultaneously represent a carbon atom and when the radical R₇ is borne by the nitrogen atom of the unsaturated ring; or alternatively
2) when at least one of the ring members E, G, J and L represents a nitrogen atom to which the radical R₇ is attached;
- in the unsaturated cationic groups of formula (III):
- when a = 0, the linker arm D is attached to the nitrogen atom,
- when a = 1, the linker arm D is attached to one of the ring members E, G, J, L or M,
- y can take the value 1 only when at least one of the ring members E, G, J, L and M represents a divalent atom and when the radical R₇ is borne by the nitrogen atom of the unsaturated ring;
- in the cationic groups of formula (IV):
- when a = 0, then the linker arm D is attached to the nitrogen atom bearing the radicals R₈ to R₁₀,
- when a = 1, then two of the radicals R₈ to R₁₀ form, together with the nitrogen atom to which they are attached, a saturated 5- or 6-membered ring as defined above, and the linker arm D is borne by a carbon atom of the said saturated ring;
• X⁻ represents a monovalent or divalent anion; it being understood that:
- the number of cationic groups Z is at least equal to 1.

2. Compounds according to Claim 1, **characterized in that** the rings of the unsaturated groups Z of formula (II) are chosen from pyrrole, imidazole, pyrazole, oxazole, thiazole and triazole rings.

3. Compounds according to Claim 1, **characterized in that** the rings of the unsaturated groups Z of formula (III) are chosen from pyridine, pyrimidine, pyrazine, oxazine and triazine rings.

4. Compounds according to any one of the preceding claims, **characterized in that** two of the radicals R₈, R₉ and R₁₀ form a pyrrolidine ring, a piperidine ring, a piperazine ring or a morpholine ring, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri (C₁-C₆) alkylsilane (C₁-C₆) alkyl radical, an amido radical, an aldehydo radical, a carboxyl radical, a keto (C₁-C₆) alkyl radical, a thio radical, a C₁-C₆ thioalkyl radical, a C₁-C₆ alkylthio radical, an amino radical or an amino radical protected with a (C₁-C₆)alkylcarbonyl, carbamyl or C₁-C₆ alkylsulphonyl radical.

5. Compounds according to any one of the preceding claims, **characterized in that** X⁻ represents a halogen atom, a hydroxide, a hydrogen sulphate or a C₁-C₆ alkyl sulphate.

6. Compounds according to any one of the preceding claims, **characterized in that** they are chosen from:
- 1-[2-(benzo[1,3]dioxol-5-ylamino)ethyl]-3-methyl-3H-imidazol-1-ium chloride;
- 1-[2-(6-hydroxybenzo[1,3]dioxol-5-ylamino)ethyl]-3-methyl-3H-imidazol-1-ium chloride;
- 1-[2-(6-methoxybenzo[1,3]dioxol-5-ylamino)ethyl]-3-methyl-3H-imidazol-1-ium chloride;
- 1-[2-(6-ethoxybenzo[1,3]dioxol-5-ylamino)ethyl]-3-methyl-3H-imidazol-1-ium chloride;
- 1-{2-[6-(2-hydroxyethoxy)benzo[1,3]dioxol-5-ylamino]ethyl}-3-methyl-3H-imidazol-1-ium chloride;
- 1-[2-(6-aminobenzo[1,3]dioxol-5-ylamino)ethyl]-3-methyl-3H-imidazol-1-ium chloride;
- 1-{2-[6-(2-(3-methyl-3H-imidazol-1-ium)ethoxy)-benzo[1,3]dioxol-5-ylamino]ethyl}-3-methyl-3H-imidazol-1-ium dichloride;
- 1-[2-(6-aminobenzo[1,3]dioxol-5-yloxy)ethyl]-3-methyl-3H-imidazol-1-ium chloride;
- 1-[3-(6-aminobenzo[1,3]dioxol-5-yloxy)propyl]-3-methyl-3H-imidazol-1-ium chloride;
- [2-(6-aminobenzo[1,3]dioxol-5-yloxy)ethyl]diethyl-(2-hydroxyethyl)ammonium bromide;
- [2-(6-aminobenzo[1,3]dioxol-5-yloxy)ethyl]diethyl-methylammonium methyl sulphate;
- 1-[2-(6-aminobenzo[1,3]dioxol-5-yloxy)ethyl]-1,4-dimethylpiperazin-1-ium chloride;
- 1-[2-(2,2-bis (hydroxymethyl)-6-methoxybenzo[1,3]-dioxol-5-ylamino)ethyl]-3-methyl-3H-imidazol-1-ium chloride;
and the addition salts thereof with an acid.

7. Compounds according to any one of the preceding claims, **characterized in that** the addition salts with an acid are chosen from the hydrochlorides, hydrobromides, sulphates, citrates, succinates, tartrates, lactates and acetates.

8. Use of the compounds of formula (I) as defined in any one of Claims 1 to 7, as oxidation dye precursors for the oxidation dyeing of keratin fibres.

9. Composition for the oxidation dyeing of keratin fibres, **characterized in that** it comprises, in a medium which is suitable for dyeing, at least one compound of formula (I) as defined in any one of Claims 1 to 7.

10. Composition according to Claim 9, **characterized in that** the compound(s) of formula (I) represent(s) from 0.0005% to 12% by weight relative to the total weight of the dye composition.

11. Composition according to Claim 10, **characterized in that** the compound(s) of formula (I) represent(s) from 0.005% to 6% by weight relative to the total weight of the dye composition.

12. Composition according to any one of Claims 9 to 11, **characterized in that** it contains one or more oxidation bases chosen from para-phenylenediamines, bis(phenyl)alkylenediamines, para-aminophenols, ortho-aminophenols and heterocyclic bases.

13. Composition according to Claim 12, **characterized in that** the heterocyclic bases are chosen from pyridine derivatives, pyrimidine derivatives and pyrazole derivatives.

14. Composition according to Claim 12 or 13,
**characterized in that** the oxidation base(s) represent(s) from 0.0005% to 12% by weight relative to the total weight of the dye composition.

15. Composition according to any one of Claims 9 to 14, **characterized in that** it contains one or more couplers chosen from meta-phenylenediamines, metaaminophenols, meta-diphenols and heterocyclic couplers, and/or one or more direct dyes.

16. Composition according to Claim 15, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)-propane, sesamol, α-naphthol, 6-hydroxyindole, 4-hydroxyindole, 4-hydroxy-N-methylindole, 6-hydroxyindoline, 2,6-dihydroxy-4-methylpyridine, 1-H-3-methylpyrazol-5-one, 1-phenyl-3-methylpyrazol-5-one and the addition salts thereof with an acid.

17. Composition according to Claim 15 or 16,
**characterized in that** the coupler(s) represent(s) from 0.0001% to 10% by weight approximately relative to the total weight of the dye composition.

18. Composition according to any one of Claims 9 to 17, **characterized in that** the addition salts with an acid are chosen from the hydrochlorides, hydrobromides, sulphates, citrates, succinates, tartrates, lactates and acetates.

19. Process for the oxidation dyeing of keratin fibres, and in particular of human keratin fibres such as the hair, **characterized in that** at least one dye composition as defined in any one of Claims 9 to 18 is applied to the fibres, for a period which is sufficient to develop the desired coloration, either in air or using an oxidizing agent.

20. Process according to Claim 19, **characterized in that** the coloration of the fibres can be carried out without adding an oxidizing agent, merely by contact with atmospheric oxygen.

21. Process according to Claim 19, **characterized in that** the colour is developed at acidic, neutral or alkaline pH using an oxidizing agent which is added to the dye composition just at the time of use, or which is present in an oxidizing composition applied simultaneously or sequentially in a separate manner.

22. Process according to Claim 21, **characterized in that** the oxidizing agent is chosen from hydrogen peroxide, urea peroxide, alkali metal bromates, persalts and enzymes.

23. Process according to Claim 22, **characterized in that** the enzymes are chosen from peroxidases, laccases, tyrosinases and oxidoreductases.

24. Process according to Claim 23, **characterized in that** the oxidoreductases are chosen from pyranose oxidases, glucose oxidases, glycerol oxidases, lactate oxidases, pyruvate oxidases and uricases.

25. Multi-compartment device or multi-compartment dyeing "kit", a first compartment of which contains a dye composition as defined in any one of Claims 9 to 18 and a second compartment of which contains an oxidizing composition.
